# EUROPEAN PATENT SPECIFICATION

(11) **EP 4 125 794 B1**
(45) Date of publication and mention of the grant of the patent: **14.02.2024**
(21) Application number: 21712551.7
(22) Date of filing: 22.03.2021
(51) Int. Cl.: A61K 8/41, A61K 8/44, A61K 8/36, A61K 8/46, A61Q 19/10

(54) **CLEANSING COMPOSITION**
REINIGUNGSMITTELZUSAMMENSETZUNG
COMPOSITION DE NETTOYAGE

(30) Priority: 31.03.2020 EP 20167051
(43) Date of publication of application: 08.02.2023
(73) Proprietor: Unilever IP Holdings B.V., 3013 AL Rotterdam (NL); Unilever Global IP Limited, Wirral, Merseyside CH62 4ZD (GB)
(72) Inventor: EARLY, James, Andrew, Trumbull, Connecticut 06611 (US); PEREIRA, Daniel, Filipe, Trumbull, Connecticut 06611 (US)
(74) Representative: Fijnvandraat, Arnoldus
(86) International application number: PCT/EP2021/057300
(87) International publication number: WO 2021/197906

(56) References cited:
- WO-A1-2016/095145
- WO-A1-2018/206215
- CN-A- 108 815 048
- DE-A1-102014 204 998
- JP-A- S61 291 035
- US-A- 4 737 360
- US-A1- 2020 085 712

## Description

### TECHNICAL FIELD

Disclosed herein is a cleansing composition. The cleansing composition comprises a fatty acid, a surfactant, and an organic buffer. The cleansing composition comprises less than 12% by weight of a C10-C18 fatty acid, 0.25 to 5% by weight of a surfactant, and 0.1 to 3% by weight of an organic buffer.

### BACKGROUND

Liquid cleansing compositions are generally very popular with consumers. The cleansing compositions are expected to foam, lather, and be rinsed from the body with relative ease. Cleansing compositions such as personal wash or shower gel compositions generally comprise fatty acids and a surfactant.

Cleansing compositions are generally formulated with various surfactants that produce large amounts of foam to clean the hair and/or skin of excess sebum and dirt. Anionic surfactants can remove natural protective oils from the skin and hair, leaving the skin and/or hair feeling dry, bristly, and/or tangled. Cleansing compositions that can provide the desired cleansing feeling and effect while utilizing lower amounts of surfactants are continually desired. Sourcing of surfactants can be costly and have an environmental impact depending on how and/or where the surfactants are sourced.

U.S. Patent Publication No. 2018/0021232 A1 to Ruan et al. discloses a combination of a salt of a short chain fatty acid together with a blend of salts of long chain fatty acids to provide excellent sensory and performance properties when formulated into a skin cleansing composition. The ratio of the amount of short chain fatty acid to the amount of blend of long chain fatty acid is from 2.5:1 to 3.5:1. The composition also has antibacterial efficacy, allowing the use of a lower dosage of antibacterial compound in the composition.

International Patent Publication No. WO 2012/078160 A1 to Fan et al. discloses an aqueous composition including (a) surfactants comprising a salt of a C10-C16 alcohol ethoxylate sulfate, a betaine surfactant, and an alkyl polyglucoside, wherein the salt of the C10-C16 alcohol ethoxylate sulfate is present in a quantity that is greater than any other surfactant; and (b) at least 15 weight % of the composition of a C12-C18 fatty acid. A cleansing method includes applying the composition to skin or hair and washing, and optionally rinsing with water.

International Patent Publication No. WO 2013/186715 A2 to Gavillon et al. discloses a rinse-off foaming cleansing cosmetic composition comprising a surfactant system in an active material of greater than or equal to 3% by weight relative to the total weight of the composition, at least one suspension agent, at least one cosmetic additive in the form of particles, fibers or mixtures thereof, and at least one water-insoluble film forming polymer in the form of particles of the type such as a copolymer comprising at least one monomer unit of acrylate type, the polymer particles having a number average primary size of greater than 500 nanometers. The invention also relates to a process for cleansing keratin materials, which consists in applying the composition to the keratin materials, in working it into a foam and then rinsing off the composition.

U.S. Patent Publication No. 2003/0134761 A1 to Sebillotte-Arnaud et al. discloses a cleansing composition comprising in a physiologically acceptable aqueous medium (1) at least one foaming surfactant, (2) at least 1% by weight of at least one silica with respect to the total weight of the composition, (3 at least one oxyalkylated compound, and (4) at least one polymer chosen from cationic polymers and amphoteric polymers. The composition has a gel consistency and gives a good lather. It can be used in cleansing products to remove makeup from the skin, eyes, scalp, and/or hair and/or to disinfect the skin and/or the scalp.

U.S. Patent Publication No. 2005/0143277 A1 to Dufay et al. discloses a surfactant composition containing (a) from about 15 to 20% by weight of an alkyl and/or alkenyl oligoglycoside; (b) from about 15 to 20% by weight of a betaine; and (c) from about 60 to 70% by weight of an alkyl ether sulfate, all weights being based on the weight of the composition.

UK Patent Application No. GB 9916322.2 to Masaaki et al. discloses a composition having a mixture of isoprene glycol and dipropylene glycol which retards freezing thereby allowing the compositions to be pumped out of their respective containers. The composition also includes soap, sodium lauryl ether sulfate, cocamido propyl betaine, glycerine, tetrasodium ethylene diaminetetraacetate (EDTA), perfume, and water. The composition is primarily used as a shower gel.

It is continually desired to provide a cleansing composition that provides desirable foaming and cleansing characteristics while at the same time reducing the surfactant concentration in the overall composition to reduce costs and more sustainably utilize natural resources.

### BRIEF DESCRIPTION OF THE DRAWINGS

The following is a brief description of the drawings wherein like elements are numbered alike and which are presented for the purposes of illustrating the cleansing compositions disclosed herein and not for the purposes of limiting the same.
FIG. 1 is a graphical illustration of the pH of various cleansing compositions versus grams of product in the final mixing solution.
FIG. 2 is a graphical illustration of the lather start of various cleansing compositions versus grams of product in the final mixing solution.
FIG. 3 is a graphical illustration of the foam volume of various cleansing compositions versus grams of product in the final mixing solution.

### BRIEF SUMMARY

Disclosed, in various aspects, are cleansing compositions. The cleansing composition can comprise a fatty acid, a surfactant, and an organic buffer. The cleansing composition comprises less than 12% by weight of a C10-C18 fatty acid. The cleansing composition comprises 0.25 to 5% by weight of a surfactant. The cleansing composition comprises 0.1 to 3% by weight of an organic buffer.

The above described and other features and characteristics are exemplified by the following detailed description.

### DETAILED DESCRIPTION

Disclosed herein is a cleansing composition. The cleansing composition can include a fatty acid, a surfactant, and an organic buffer. The fatty acid can comprise a C10-C18 fatty acid. The cleansing composition can comprise less than 12% by weight of the fatty acid. The cleansing composition can comprise 0.25 to 5% by weight of a surfactant. The composition can comprise 0.1 to 3% by weight of an organic buffer. Previously, a reduction in the amount of surfactant present in a composition resulted in a reduction in foam characteristics and/or performance. The cleansing composition disclosed herein can allow for a reduced level of surfactant in the composition with no loss of desirable cleansing properties such as foaming, cleansing, and/or rinsing. The cleansing composition disclosed herein can also provide for enhanced or even instant foaming effects. Without wishing to be bound by theory, it is believed that dilution of cleansing compositions while in use (e.g., such as in a shower) causes a reversion of the cleansing composition to its individual components due to a lack of available counter ions. For example, the cleansing composition reverts back to its fatty acid component because of the lack of available counter ions. It was unexpectedly found that the inclusion of an organic buffer in the cleansing composition provided a reservoir of counter ions to associate with the fatty acid in the dilute environment thereby maintaining the levels of fatty acid and surfactant present in the cleansing composition. The inclusion of an organic buffer also allowed for no loss in foam performance as compared to a cleansing composition with a higher amount of surfactant. The cleansing composition disclosed herein reduces the amount of total fatty acid present in the composition and the amount of overall surfactant present in the composition while maintaining foaming, cleansing, and/or rinsing performance.

It was unexpectedly found that the inclusion of an organic buffer allowed the pH of the cleansing composition to be adjusted resulting in a desirable amount and rate of foaming even with a lower amount of surfactant present in the cleansing composition. Stated another way, there was a synergistic effect between the organic buffer and the surfactant which allowed the lather time to remain the same or slightly less as compared to a cleansing composition with an increased amount of surfactant. The synergistic effect also provides the benefit of desired foaming in the cleansing composition. The organic buffer can provide ions to replace those ions that are lost during initial neutralization of the fatty acid. Replacing the lost ions can assist in maintaining constant pH and surfactant levels of the cleansing composition. It was surprisingly found that the pH of the cleansing composition actually increased upon dilution in the non-buffered systems, when the organic buffer was added to the cleansing composition the pH trend was corrected, where the pH decreased during dilution. It was surprising that the amount of foaming even increased with the inclusion of an organic buffer in the cleansing composition. The amount of surfactant used in the cleansing composition can be reduced by less than or equal to 25%, for example, less than or equal to 50%, for example, less than or equal to 40, for example, less than or equal to 30%, for example, less than or equal to 20%, for example, less than or equal to 15%, for example, less than or equal to 10%. The pH of the cleansing composition can be 8.0 to 10.0, preferably, 8.5 to 10.0, more preferably, 9.0 to 10.0.

A molar ratio of the organic buffer to the surfactant can be 1:5 to 3:2, preferably, 1:4 to 1:1, more preferably, 1:4 to 1:2. A molar ratio of the organic buffer to total fatty acid in the cleansing composition can be 1.2 to 1.5, preferably, 1.15 to 1.7, more preferably, 1.12 to 1.10.

The fatty acid of the cleansing composition can be present in an amount of less than 20% by weight. For example, the fatty acid can be present in an amount of less than 16%, by weight, for example, less than 13% by weight, for example, less than 12% by weight, for example, 5% by weight to 20% by weight, for example, 7.5% by weight to 15% by weight, for example, 9% by weight to 12% by weight, for example, 10% by weight to 11.5 % by weight.

The fatty acid contains at least 10 carbon atoms, preferably at least 12 carbon atoms. The fatty acid can contain 10 to 18 carbon atoms, preferably, 12 to 16 carbon atoms. The fatty acids can be saturated, unsaturated, straight chain, or branched. The fatty acid can be selected from lauric acid, myristic acid, palmitic acid, stearic acid, behenic acid, oleic acid, linoleic acid, lanolic acid, isostearic acid, arachidonic acid, hydroxystearic acid, or a combination thereof.

The fatty acid can be neutralized with a neutralizing agent to form soap. For example, the fatty acid can be neutralized with an oxide. The oxide can include, but is not limited to, sodium hydroxide (NaOH), potassium hydroxide (KOH), sodium carbonate (Na₂CO₃), ammonium hydroxide (NH₄OH) or a combination thereof. In an aspect, the fatty acid is neutralized with sodium hydroxide or potassium hydroxide. In a preferred aspect, the fatty acid is neutralized with potassium hydroxide.

The surfactant of the cleansing composition can be present in an amount of 0.25 to 5% by weight. For example, the surfactant can be present in an amount of 0.5 to 3.5% by weight, for example, 0.75 to 2.0% by weight, for example, 1.0 to 1.5% by weight, for example 1.1 to 1.4% by weight.

The surfactant can be selected from an anionic surfactant, a zwitterionic surfactant, an amphoteric surfactant, or a combination thereof.

As to the anionic surfactant present in the cleansing composition disclosed herein, the anionic surfactant used can include aliphatic sulfonates, such as a primary alkane (e.g., C₈-C₂₂) sulfonate, primary alkane (e.g., C₈-C₂₂) disulfonate, C₈-C₂₂ alkene sulfonate, C₈-C₂₂ hydroxyalkane sulfonate or alkyl glyceryl ether sulfonate (AGS); or aromatic sulfonates such as alkyl benzene sulfonate. The anionic may also be an alkyl sulfate (e.g., C₁₂-C₁₈ alkyl sulfate) or alkyl ether sulfate (including alkyl glyceryl ether sulfates). Among the alkyl ether sulfates are those having the formula:

RO(CH₂CH₂O)ₙSO₃M

wherein R is an alkyl or alkenyl having 8 to 18 carbons, preferably 12 to 18 carbons, n has an average value of at least 1.0, preferably less than 5, and most preferably 1 to 4, and M is a solubilizing cation such as sodium, potassium, ammonium or substituted ammonium.

The anionic surfactant may also be alkyl sulfosuccinates (including mono- and dialkyl, e.g., C₆-C₂₂ sulfosuccinates); alkyl and acyl taurates (often methyl taurates), alkyl and acyl sarcosinates, sulfoacetates, C₈-C₂₂ alkyl phosphates and phosphonates, alkyl phosphate esters and alkoxyl alkyl phosphate esters, acyl lactates, C₈-C₂₂ monoalkyl succinates and maleates, sulphoacetates, alkyl glucosides and acyl isethionates, and the like.

Sulfosuccinates may be monoalkyl sulfosuccinates having the formula:

R¹O₂CCH₂CH(SO₃M)CO₂M;

and amide-MEA sulfosuccinates of the formula:

R¹CONHCH₂CH₂O₂CCH₂CH(SO₃M)CO₂M

wherein R¹ ranges from C₈-C₂₂ alkyl.

Sarcosinates are generally indicated by the formula:
R²CON(CH₃)CH₂CO₂M, wherein R² ranges from C₈-C₂₀ alkyl.

Taurates are generally identified by formula:

R³CONR⁴CH₂CH₂SO₃M

wherein R³ is a C₈-C₂₀ alkyl, R⁴ is a C₁-C₄ alkyl.

M is a solubilizing cation as previously described.

The cleansing composition disclosed herein may contain C₈-C₁₈ acyl isethionates. These esters are prepared by a reaction between alkali metal isethionate with mixed aliphatic fatty acids having from 6 to 18 carbon atoms and an iodine value of less than 20. At least 75% of the mixed fatty acids have from 12 to 18 carbon atoms and up to 25% have from 6 to 10 carbon atoms.

The acyl isethionate may be an alkoxylated isethionate such as is described in Ilardi et al., U.S. Pat. No. 5,393,466, entitled "Fatty Acid Esters of Polyalkoxylated isethonic acid; issued Feb. 28, 1995. This compound has the general formula:

R⁵C-(O)O-C(X)H-C(Y)H-(OCH₂-CH₂)ₘ-SO₃M

wherein R⁵ is an alkyl group having 8 to 18 carbons, m is an integer from 1 to 4, X and Y are each independently hydrogen or an alkyl group having 1 to 4 carbons and M is a solubilizing cation as previously described.

In an aspect of the cleansing composition, the anionic surfactant used is 2-acrylamido-2-methylpropane sulfonic acid, ammonium lauryl sulfate, ammonium perfluorononanoate, potassium lauryl sulfate, sodium alkyl sulfate, sodium dodecyl sulfate, sodium laurate, sodium laureth sulfate, sodium lauroyl sarcosinate, sodium stearate, sodium sulfosuccinate esters, or a combination thereof. Such anionic surfactants are commercially available from suppliers like Galaxy Surfactants, Clariant, Sino Lion, Stepan Company, and Innospec.

Optionally, amphoteric surfactants can be included in the cleansing compositions disclosed herein. Amphoteric surfactants (which depending on pH can be zwitterionic) include sodium acyl amphoacetates, sodium acyl amphopropionates, disodium acyl amphodiacetates and disodium acyl amphodipropionates where the acyl (i.e., alkanoyl group) can comprise a C₇-C₁₈ alkyl portion. Illustrative examples of amphoteric surfactants include sodium lauroamphoacetate, sodium cocoamphoacetate, sodium lauroamphoacetate, sodium cocoamphoacetate and or a combination thereof.

The zwitterionic surfactant disclosed herein can be present in the cleansing composition in an amount of 3 to 10% by weight, preferably 4 to 8% by weight, more preferably 5 to 7% by weight. In an aspect, the zwitterionic surfactant is present in an amount of greater than 5% by weight. As to the zwitterionic surfactants employed in the present cleansing composition, such surfactants include at least one acid group. Such an acid group may be a carboxylic or a sulphonic acid group. They often include quaternary nitrogen, and therefore, can be quaternary amino acids. They should generally include an alkyl or alkenyl group of 7 to 18 carbon atoms generally comply with an overall structural formula:

R⁶-[-C(O)-NH(CH₂)_{q}-]ᵣ-N⁺(R⁷)(R⁸)-A-B

where R⁷ is alkyl or alkenyl of 7 to 18 carbon atoms; R⁷ and R⁸ are each independently alkyl, hydroxyalkyl or carboxyalkyl of 1 to 3 carbon atoms; q is 2 to 4; r is 0 to 1; A is alkylene of 1 to 3 carbon atoms optionally substituted with hydroxyl, and B is -CO₂- or -SO₃-.

Desirable zwitterionic surfactants for use in the cleansing composition disclosed herein and within the above general formula include simple betaines of formula:

R⁶-N⁺(R⁷)(R⁸)-CH₂CO₂⁻

and amido betaines of formula:

R⁶-CONH(CH₂)ₜ-N⁺ (R⁷)(R⁸)-CH₂CO₂⁻

where t is 2 or 3.

In both formulae R⁶, R⁷ and R⁸ are as defined previously. R⁶ may, in particular, be a mixture of C₁₂ and C₁₄ alkyl groups derived from coconut oil so that at least half, preferably at least three quarters of the groups R⁶ have 10 to 14 carbon atoms. R⁷ and R⁸ are preferably methyl.

A further possibility is that the zwitterionic surfactant is a sulphobetaine of formula:

R⁶-N⁺(R⁷)(R⁸)-(CH₂)₃SO₃⁻

or

R⁶-CONH(CH₂)ᵤ-N⁺(R⁷)(R⁸)-(CH₂)₃SO₃⁻

where u is 2 or 3, or variants of these in which --(CH₂)₃SO₃⁻ is replaced by - CH₂C(OH)(H)CH₂SO₃⁻.

In these formulae, R⁶, R⁷ and R⁸ are as previously defined.

Illustrative examples of the zwitterionic surfactants desirable for use include betaines such as lauryl betaine, betaine citrate, cocodimethyl carboxymethyl betaine, cocoamidopropyl betaine, coco alkyldimethyl betaine, and laurylamidopropyl betaine. Other zwitterionic surfactants desirable for use include, but are not limited to, An additional zwitterionic surfactant suitable for use includes cocoamidopropyl sultaine, for example, cocamidopropyl hydroxysultaine. Preferred zwitterionic surfactants include lauryl betaine, betaine citrate, sodium hydroxymethylglycinate, carboxymethyl)dimethyl-3-[(1-oxododecyl) amino] propylammonium hydroxide, coco alkyldimethyl betaine, (carboxymethyl) dimethyloleylammonium hydroxide, cocoamidopropyl betaine, (carboxymethyl) dimethyloleylammonium hydroxide, cocoamidopropyl betaine, (carboxylatomethyl) dimethyl(octadecyl)ammonium, cocamidopropyl hydroxysultaine, or a combination thereof. Such surfactants are made commercially available from suppliers like Stepan Company, Solvay, Evonik and the like and it is within the scope of the cleansing compositions disclosed herein to employ mixtures of the aforementioned surfactants.

Nonionic surfactants may optionally be used in the cleansing composition. When used, nonionic surfactants are typically used at levels as low as 0.5, 1, 1.5 or 2% by weight and at levels as high as 6, 8, 10 or 12% by weight. The nonionics which may be used include in particular the reaction products of compounds having a hydrophobic group and a reactive hydrogen atom, for example aliphatic alcohols, acids, amides or alkylphenols with alkylene oxides, especially ethylene oxide either alone or with propylene oxide. Specific nonionic surfactant compounds are alkyl (C₆-C₂₂) phenols ethylene oxide condensates, the condensation products of aliphatic (C₈-C₁₈) primary or secondary linear or branched alcohols with ethylene oxide, and products made by condensation of ethylene oxide with the reaction products of propylene oxide and ethylenediamine. Other nonionic surfactants include long chain tertiary amine oxides, long chain tertiary phosphine oxides, dialkyl sulphoxides, and the like.

In an aspect, nonionic surfactants can include fatty acid/alcohol ethoxylates having the following structures a) HOCH₂(CH₂)ₛ(CH₂CH₂O)ᵥ H or b) HOOC(CH₂)_{c}(CH₂CH₂O)_{d} H; where s and v are each independently an integer up to 18; and c and d are each independently an integer from 1 or greater. In an aspect, s and v can be each independently 6 to 18; and c and d can be each independently 1 to 30. Other options for nonionic surfactants include those having the formula HOOC(CH₂)ᵢ-CH=CH-(CH₂)ₖ(CH₂CH₂O)_{z} H, where i, k are each independently 5 to 15; and z is 5 to 50. In another aspect, i and k are each independently 6 to 12; and z is 15 to 35.

The nonionic surfactant may also include a sugar amide, such as a polysaccharide amide. Specifically, the surfactant may be one of the lactobionamides described in U.S. Pat. No. 5,389,279 to Au et al., entitled "Compositions Comprising Nonionic Glycolipid Surfactants issued Feb. 14, 1995, or it may be one of the sugar amides described in U.S. Pat. No. 5,009,814 to Kelkenberg, titled "Use of N-Poly Hydroxyalkyl Fatty Acid Amides as Thickening Agents for Liquid Aqueous Surfactant Systems" issued Apr. 23, 1991.

Illustrative examples of nonionic surfactants that can optionally be used in the cleansing compositions disclosed herein include, but are not limited to, polyglycoside, cetyl alcohol, decyl glucoside, lauryl glucoside, octaethylene glycol monododecyl ether, n-octyl beta-d-thioglucopyranoside, octyl glucoside, oleyl alcohol, polysorbate, sorbitan, stearyl alcohol, or a combination thereof.

In an aspect, cationic surfactants may optionally be used in the cleansing composition of the present application.

One class of cationic surfactants includes heterocyclic ammonium salts such as cetyl or stearyl pyridinium chloride, alkyl amidoethyl pyrrylinodium methyl sulfate, and lapyrium chloride.

Tetra alkyl ammonium salts are another useful class of cationic surfactants suitable for use. Examples include cetyl or stearyl trimethyl ammonium chloride or bromide; hydrogenated palm or tallow trimethylammonium halides; behenyl trimethyl ammonium halides or methyl sulfates; decyl isononyl dimethyl ammonium halides; ditallow (or distearyl) dimethyl ammonium halides, and behenyl dimethyl ammonium chloride.

Still other types of cationic surfactants that may be used are the various ethoxylated quaternary amines and ester quats. Examples include PEG-5 stearyl ammonium lactate (e.g., Genamin KSL manufactured by Clariant), PEG-2 coco ammonium chloride, PEG-15 hydrogenated tallow ammonium chloride, PEG 15 stearyl ammonium chloride, dipalmitoyl ethyl methyl ammonium chloride, dipalmitoyl hydroxyethyl methyl sulfate, and strearyl amidopropyl dimethylamine lactate.

Still other useful cationic surfactants include quaternized hydrolysates of silk, wheat, and keratin proteins, and it is within the scope of the cleansing composition to use mixtures of the aforementioned cationic surfactants.

If used, cationic surfactants will make up no more than 1.0% by weight of the cleansing composition. When present, cationic surfactants typically make up from 0.01 to 0.7%, and more typically, from 0.1 to 0.5% by weight of the cleansing composition, including all ranges subsumed therein.

The cleansing composition also includes an organic buffer. The organic buffer can be present in an amount of 0.1 to 3% by weight in the cleansing composition, for example, 0.25 to 2% by weight, for example, 0.5 to 1.5% by weight, for example, 0.7 to 1.0% by weight, for example, 0.1 to 0.7% by weight. The organic buffer can comprise an amine, a salt, or a combination thereof. For example, the organic buffer can comprise triethanolamine, diethanolamine, triethanolamine sodium phosphate, calcium carbonate, sodium phosphate, disodium phosphate, trisodium phosphate, or a combination thereof. In an aspect, the organic buffer can comprise triethanolamine. The organic buffer can have a pKa range of 6.0 to 9.0, preferably, 6.5 to 8.5, more preferably, 7.0 to 8.0.

The cleansing composition disclosed herein can further include a thickening agent in an amount of 0.5 to 2.5% by weight, preferably 0.75 to 2.0% by weight, more preferably 1.0 to 1.5% by weight. Particularly useful are the polysaccharides. Examples include fibers, starches, natural/synthetic gums and cellulosics. Representative of the starches are chemically modified starches such as sodium hydroxypropyl starch phosphate and aluminum starch octenylsuccinate. Tapioca starch is often preferred, as is maltodextrin. Suitable gums include xanthan, sclerotium, pectin, karaya, arabic, agar, guar (including Acacia senegal guar), carrageenan, alginate and combinations thereof. Suitable cellulosics include hydroxypropyl cellulose, hydroxypropyl methylcellulose, ethylcellulose, sodium carboxy methylcellulose (cellulose gum/carboxymethyl cellulose) and cellulose (e.g. cellulose microfibrils, cellulose nanocrystals or microcrystalline cellulose).

Sources of cellulose microfibrils include secondary cell wall materials (e.g. wood pulp, cotton), bacterial cellulose, and primary cell wall materials. Preferably the source of primary cell wall material is selected from parenchymal tissue from fruits, roots, bulbs, tubers, seeds, leaves and combination thereof; more preferably is selected from citrus fruit, tomato fruit, peach fruit, pumpkin fruit, kiwi fruit, apple fruit, mango fruit, sugar beet, beet root, turnip, parsnip, maize, oat, wheat, peas and combinations thereof; and even more preferably is selected from citrus fruit, tomato fruit and combinations thereof. A most preferred source of primary cell wall material is parenchymal tissue from citrus fruit. Citrus fibers, such as those made available by Herbacel^{®} as AQ Plus can also be used as source for cellulose microfibrils. The cellulose sources can be surface modified by any of the known methods including those described in Colloidal Polymer Science, Kalia et al., "Nanofibrillated cellulose: surface modification and potential applications" (2014), Vol 292, Pages 5-31.

Synthetic polymers are yet another class of effective thickening agent. This category includes crosslinked polyacrylates such as the Carbomers, polyacrylamides such as Sepigel^{®} 305 and taurate copolymers such as Simulgel^{®} EG and Aristoflex^{®} AVC, the copolymers being identified by respective INCI nomenclature as Sodium Acrylate/Sodium Acryloyldimethyl Taurate and Acryloyl Dimethyltaurate/Vinyl Pyrrolidone Copolymer. Another preferred synthetic polymer suitable for thickening is an acrylate-based polymer made commercially available by Seppic and sold under the name Simulgel INS100. Calcium carbonate, fumed silica, and magnesium-aluminum-silicate may also be used.

Particularly preferred thickening agents include sodium hydroxypropyl starch phosphate, aluminum starch octenylsuccinate, tapioca starch, maltodextrin, xanthan gum, agar gum, guar gum, carrageenan gum, alginate gum, hydroxypropyl cellulose, hydroxypropyl methylcellulose, ethylcellulose, sodium carboxy methylcellulose, cellulose, polyethylene glycol (e.g., polyethylene glycol diester stearic acid), or a combination thereof. Such thickening agents are commercially available from the Dow Chemical Company or the Hallstar Company.

The cleansing composition can further optionally include an anti-static agent. The anti-static agent can include imidazolinium salt, pyridinium salt, piperidinium salt, morpholinium salts, quaternary ammonium salt, or a combination thereof. For example, the anti-static agent can be a polyquaternium salt. One such polyquaternium salt is polyquaternium 10 available from various suppliers including KCL Limited and the Dow Chemical Company. When present, the anti-static agent can be present in an amount of less than 1% by weight, preferably, less than 0.5% by weight, more preferably, less than 0.25% by weight. For example, the anti-static agent can be present in an amount of 0.01 to 0.2% by weight, for example, 0.1% by weight.

A conditioning agent can optionally be included in the cleansing composition. Conditioning agents can include occlusives, e.g., petrolatum, dimethicone, and the like; humectants, e.g., glycerin, propylene glycol, sorbitol, and the like; emollients and oils, e.g., trigylcerides, natural oils, lanolin, synthetic esters, and the like; proteins; silicones, e.g., dimethicone, cyclomethicone, amodimethicone, and the like; cationic surfactants, e.g., cetrimonium chloride, stearalkonium chloride, and the like; and polymers, e.g., cationic polymers such as polyquarterniums. When present, the conditioning agent can be present in an amount of 2% by weight to 7% by weight, preferably, 3% by weight to 6% by weight, more preferably, 3.5% by weight to 5% by weight.

Water preferably makes up 10 to 99% by weight of the liquid and composition, preferably 65 to 95% by weight of the liquid and composition, and more preferably, from 70 to 90% by weight water based on total weight of the liquid and composition, including all ranges subsumed therein.

Preservatives can desirably be incorporated into the foam precursor liquid and foam cleansing composition to protect against the growth of potentially harmful microorganisms. Cosmetic chemists are familiar with appropriate preservatives and routinely choose them to satisfy the preservative challenge test and to provide product stability. Traditional preservatives for use include hydantoin derivatives and propionate salts. Preservatives for use are iodopropynyl butyl carbamate, phenoxyethanol, 1,2-octanediol, hydroxyacetophenone, ethylhexylglycerine, hexylene glycol, methyl paraben, propyl paraben, imidazolidinyl urea, sodium dehydroacetate, dimethyl-dimethyl (DMDM) hydantoin and benzyl alcohol and mixtures thereof. Other preservatives include sodium benzoate, sodium dehydroacetate, chlorophenesin and decylene glycol. Other preservatives include trisodium ethylenediamine disccinate (EDDS), tetra sodium iminodicusscinate (IDS), sodium gluconate, phytic acid, sodium phytate, tricalcium citrate, trisodium dicarboxymethyl alaninate, caproyl/capryloyl anhydro methyl glucamide and water, caprylyl glycol, ethyl lauroyl arginate hydrochloric acid and glycerin, ethyl lauroyl arginate hydrochloric acid and capryl glycol and glycerin, gluconolactone, glyceryl caprylate, lactic acid, p-anisic acid, pentylene glycol, sodium citrate, sorbitan caprylate, butylated hydroxytoluene (BHT), dilauryl thiodipropionate, octadecyl di-t-butyl-4-hydroxyhydrocinnamate, pentaerythrityl tetra-dit-butyll hydrohydrocinnamate, vitamin E, or a combination thereof. The preservatives should be selected having regard for the use of the composition and possible incompatibilities between the preservatives and other ingredients in the emulsion. Preservatives are preferably employed in amounts ranging from 0.01% to 2% by weight of the total weight of the composition, including all ranges subsumed therein. Also preferred is a preservative system with hydroxyacetophenone alone or in a mixture with other preservatives. Particularly preferred is sodium benzoate. Other preservatives that can be used include benzoin gum, sapindus mukorossi (soap nut) fruit extract, or a combination thereon.

The cleansing composition can additionally include various additives including, but not limited to, colorants, emollients, anti-dandruff agents, skin feel agents, hair dyes, styling polymer, silicon oil, cationic polymers, or a combination thereof. Each of these substances may range from about 0.03 to about 5%, preferably between 0.1 and 3% by weight of the total weight of the liquid and composition, including all ranges subsumed therein. For example, colorants can be present in an amount of 5 parts per million (ppm) to 15 ppm, for example, about 15 ppm.

Fragrances, fixatives, chelators (like EDTA) salts (like NaCl) and exfoliants may optionally be included in the liquid and composition disclosed herein. Each of these substances may range from about 0.03 to about 5%, preferably between 0.1 and 3% by weight of the total weight of the liquid and composition, including all ranges subsumed therein. For example, a chelating agent such as disodium EDTA can be present in an amount of 0.05% by weight.

Other additives that can optionally be present in the cleansing composition include viscosity adjusting agents. Viscosity adjusting agents, when present, can be present in an amount of 0.5 to 2.5% by weight, preferably, 0.75 to 1.5% by weight, more preferably 1.25 to 1.4% by weight. Viscosity adjusting agents can include those commonly known such as sodium chloride, potassium chloride, or glycols such as polypropylene glycol, e.g., PPG-9.

The cleansing composition can have a dissolution time of 1 to 5000 seconds, preferably, 1 to 10 seconds, more preferably, 1 to 5 seconds. The compositions can include dissolution rates suitable for use as a personal wash or hair cleansing product.

Cleansing and/or conditioning hair can be accomplished with the cleansing composition disclosed herein. Cleansing skin can be accomplished with the cleansing composition disclosed herein. The cleansing composition of the present application can be used to cleanse a body and/or condition hair by applying the cleansing composition to hair or skin. The cleansing composition of the present application can be used in a cleansing product, including, but not limited to, body wash, shampoo, and/or conditioner.

The cleansing composition disclosed herein can be poured into the hands of the person using it. The cleansing composition can be dissolved in water to form a thick and rich foaming lather for the skin and/or hair.

Methods of making the cleansing composition disclosed herein are also contemplated. A method can include forming the cleansing composition as described herein Forming the cleansing composition can include charging cellulosic polymer to the initial water phase. Heating the water phase to 65 °C and the fatty acids are added. At 65 °C, the fatty acids are neutralized to form the conjugate ion soap after which salts and buffers are added and the mixture cooled. During cooling, syndets are added and when near room temperature, fragrance, visual ingredients, and actives are added. After the composition is formed, it can be agitated for a period of time sufficient to form a foam. For example, the period of time for agitation can be less than or equal to 5 minutes, for example, less than or equal to 4 minutes, for example, less than or equal to 3 minutes. In an aspect, the cleansing composition can form a measurable amount of lather within 4 seconds.

The Examples provided are to facilitate an understanding of the cleansing composition. The Examples are not intended to limit the scope of the claims.

### EXAMPLE I

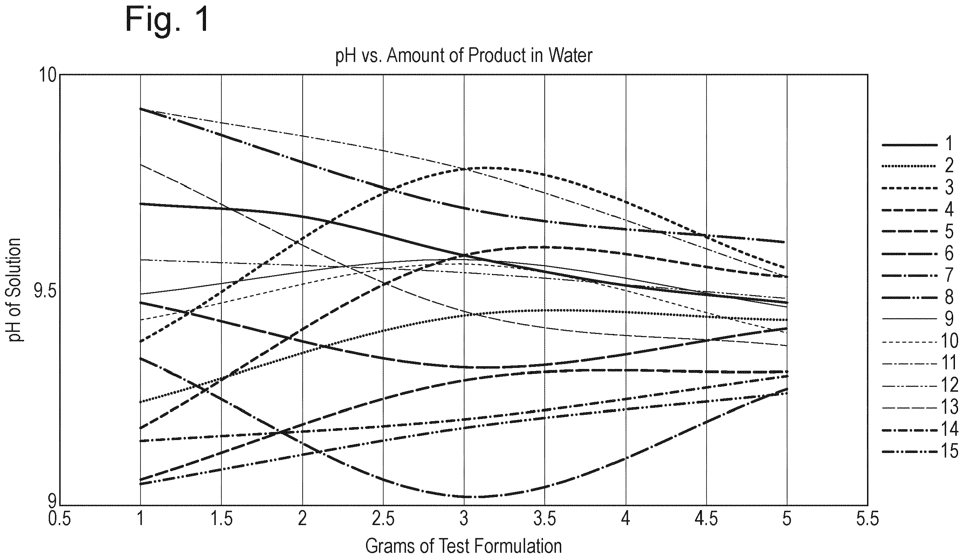

The cleansing compositions are prepared by mixing using the method disclosed herein with the amounts of various components as listed in Table 1. Once the cleansing compositions were made, three inclusion levels were evaluated, 1 milliliter (ml), 3 ml, and 5 ml in a Kruss DFA foam analyzer The compositions comprised various mixtures of palmitic acid (PA), lauric acid (LA), mysteric acid (MA), sodium lauryl ether sulfate (SLES), alkyl polyglucoside (APG), cocamidopropyl betaine (CAPB), and triethanolamine (TEA). Water was added until the 55 milliliter (mL) mark was reached. The water added was 37 °C and the source was tap water. The sample was agitated for 3 minutes and foam generation recorded by Via closed camera imaging and light transmission through the sample. Following the foam evaluation, the sample pH was measured with a standard laboratory pH meter. Data from the foam generation was evaluated and is shown in Figures 1 to 3. Foam volume was measured via light transmission through the sample to record the liquid height and subtracting it from the overall (liquid and foam) height.

**Table 1**

| Sample # | PA (g) | LA (g) | MA (g) | Total fatty acid (g) | SLES (g) | APG (g) | CAPB(g) | Total surfactant (g) | TEA (g) |
|---|---|---|---|---|---|---|---|---|---|
| 1 | 2.14 | 5.84 | 6.66 | 14.64 | 2.14 | 0 | 0.71 | 2.85 | 0 |
| 2 | 2.14 | 4.17 | 4.76 | 11.07 | 4.29 | 0 | 4.28 | 8.57 | 1.77 |
| 3 | 2.14 | 4.17 | 4.76 | 11.07 | 6.43 | 0 | 2.14 | 8.57 | 3.55 |
| 4 | 2.14 | 4.17 | 4.76 | 11.07 | 2.14 | 0 | 6.41 | 8.55 | 3.55 |
| 5 | 2.14 | 5.84 | 6.66 | 14.64 | 0.71 | 0 | 2.14 | 2.85 | 3.55 |
| 6 | 2.14 | 5.01 | 5.71 | 12.86 | 2.86 | 0 | 2.85 | 5.71 | 0 |
| 7 | 2.14 | 4.17 | 4.76 | 11.07 | 2.14 | 0 | 6.41 | 8.55 | 0 |
| 8 | 2.14 | 5.84 | 6.66 | 14.64 | 0.71 | 0 | 2.14 | 2.85 | 0 |
| 9 | 2.14 | 5.01 | 5.71 | 12.86 | 4.29 | 0 | 1.43 | 5.72 | 1.77 |
| 10 | 2.14 | 4.17 | 4.76 | 11.07 | 6.43 | 0 | 2.14 | 8.57 | 0 |
| 11 | 2.14 | 5.84 | 6.66 | 14.64 | 2.14 | 0 | 0.71 | 2.85 | 0 |
| 12 | 2.14 | 5.01 | 5.71 | 12.86 | 1.43 | 0 | 4.28 | 5.71 | 1.77 |
| 13 | 2.14 | 5.84 | 6.66 | 14.64 | 2.14 | 0 | 0.71 | 2.85 | 3.55 |

As shown in Table 1 and Figure 1, in Sample 4, the slope of the pH is inverse compared to what would be expected. Samples 1 to 13 all show a large variability in the pH slope as seen in Figure 1. The negative pH slope seen in the un-buffered samples was corrected with the addition of TEA, these buffered samples showed a notable flattening, if not inversion of the pH slope, reflecting the pH response expected.

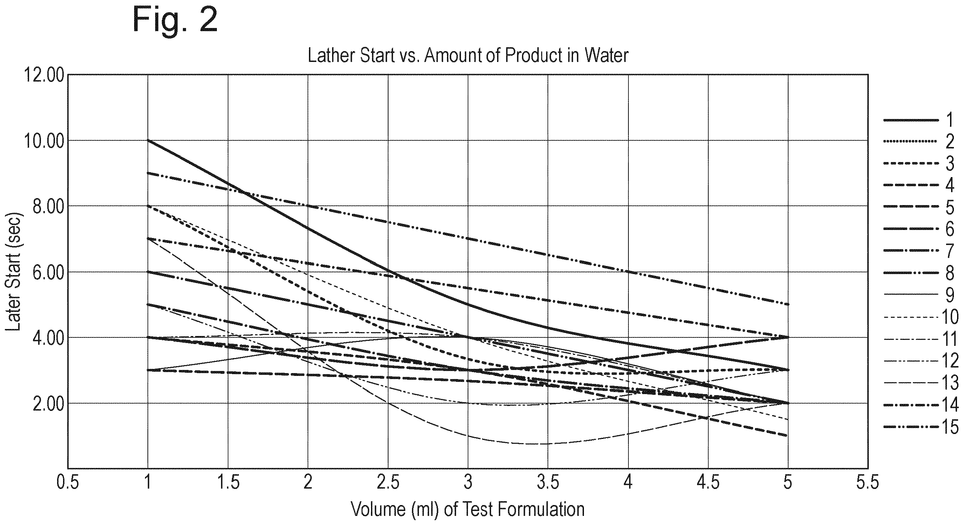

Also, in Sample 4 there is a marked improvement in the lather start time as noted in the flatted slope in Figure 2. Additionally, a marked increase in total foam volume was noted in this sample, which exhibits an independence from the sample amount, overcoming what is referred to as a "dosing penalty".

### EXAMPLE II

**Table 1**

| Sample # | PA (g) | LA (g) | MA (g) | Total fatty acid (g) | SLES (g) | APG (g) | CAPB(g) | Total surfactant (g) | TEA (g) |
|---|---|---|---|---|---|---|---|---|---|
| 14 | 1.71 | 4.67 | 5.33 | 11.71 | 0.565 | 0 | 0.565 | 1.130 | 0.71 |
| 15 | 1.71 | 4.67 | 5.33 | 11.71 | 0 | 0.246 | 0.846 | 1.092 | 0.71 |

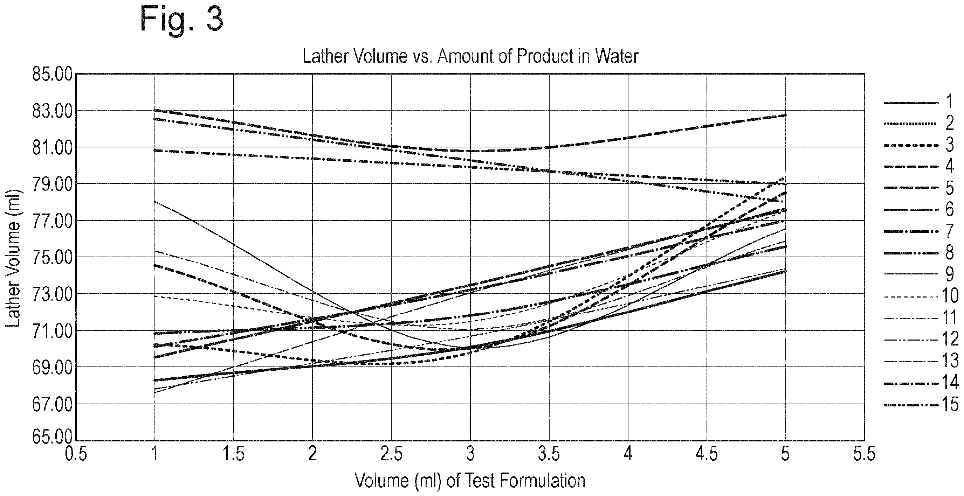

Samples 14 and 15 contained less total surfactant as compared to Samples 1 to 12. It was surprisingly discovered that in Samples 14 and 15, the pH of the composition decreased with a dosage reduction in the amount of surfactant present in the composition. This is illustrated in Figure 1, where Samples 14 and 15 had a correspondingly increasing pH relative to the volume of product. Stated another way, Samples 14 and 15 demonstrated less drift in the pH as compared to Samples 1 to 13. It was discovered that the samples containing the organic buffer showed some form of correction in pH versus dose, but in Samples 14 and 15 the pH continuously increased even with the reduction in overall surfactant present in the composition. Figure 2 shows that lather start is dependent on the amount of surfactant present in the composition. Lather start was measured in seconds. In Figure 3, foam volume is shown versus volume of product used where foam volume is measured in ml. As can be seen from Figure 3, lather foam volume is dependent on the amount of surfactant present in the composition.

All amounts are by weight of the final composition, unless otherwise specified.

It should be noted that in specifying any range of concentration or amount, any particular upper concentration can be associated with any particular lower concentration or amount. In that regard, it is noted that all ranges disclosed herein are inclusive of the endpoints, and the endpoints are independently combinable with each other (e.g., ranges of "up to 25% by weight, or, more specifically, 5% by weight to 20% by weight, in inclusive of the endpoints and all intermediate values of the ranges of 5% by weight to 25% by weight, etc.). "Combination is inclusive of blends, mixtures, alloys, reaction products, and the like. Furthermore, the terms "first", "second", and the like herein do not denote any order, quantity, or importance, but rather are used to distinguish one element from another. The terms "a" and "an" and "the" herein do not denote a limitation of quantity and are to be construed to cover both the singular and the plural, unless otherwise indicated herein or clearly contradicted by context. The suffix "(s)" as used herein is intended to include both the singular and the plural of the term if modifies, thereby including one or more of the term (e.g., the film(s) includes one or more films). Reference throughout the specification to "one embodiment", "one aspect", "another embodiment", "another aspect", "an embodiment", "an aspect" and so forth means that a particular element (e.g., feature, structure, and/or characteristic) described in connection with the embodiment or aspect is included in at least one embodiment or aspect described herein and may or may not be present in other embodiments or aspects.

## Claims

1. A cleansing composition, comprising:
less than 12% by weight of a C10-C18 fatty acid;
0.25 to 5% by weight of a surfactant; and
0.1 to 3% by weight of an organic buffer;
wherein a pH of the cleansing composition is 8.0 to 10.0, preferably 8.5 to 10.0, more preferably, 9.0 to 10.0.

2. The cleansing composition of Claim 1, wherein the fatty acid is selected from lauric acid, myristic acid, palmitic acid, stearic acid, behenic acid, oleic acid, linoleic acid, lanolic acid, isostearic acid, arachidonic acid, hydroxystearic acid, or a combination thereof.

3. The cleansing composition of Claim 1 or Claim 2, wherein the surfactant is selected from an anionic surfactant, a zwitterionic surfactant, an amphoteric surfactant, or a combination thereof.

4. The cleansing composition of Claim 3, wherein the anionic surfactant is selected from 2-acrylamido-2-methylpropane sulfonic acid, ammonium lauryl sulfate, ammonium perfluorononanoate, potassium lauryl sulfate, sodium alkyl sulfate, sodium dodecyl sulfate, sodium laurate, sodium laureth sulfate, sodium lauroyl sarcosinate, sodium stearate, sodium sulfosuccinate esters, or a combination thereof.

5. The cleansing composition of Claim 3, wherein the zwitterionic surfactant is selected from lauryl betaine, betaine citrate, sodium hydroxymethylglycinate, carboxymethyl)dimethyl-3-[(1-oxododecyl) amino] propylammonium hydroxide, coco alkyldimethyl betaines, (carboxymethyl) dimethyloleylammonium hydroxide, cocoamidopropyl betaine,
(carboxylatomethyl) dimethyl(octadecyl)ammonium, cocamidopropyl hydroxysultaine, or a combination thereof or wherein the amphoteric surfactant is selected from sodium lauroamphoacetate, sodium cocoamphoacetate, sodium lauroamphoacetate, sodium cocoamphoacetate and or a combination thereof.

6. The cleansing composition of Claim 3, wherein the surfactant further optionally comprises a nonionic surfactant, wherein the nonionic surfactant is selected from alkyl polyglycoside, cetyl alcohol, decyl glucoside, lauryl glucoside, octaethylene glycol monododecyl ether, n-octyl beta-d-thioglucopyranoside, octyl glucoside, oleyl alcohol, polysorbate, sorbitan, stearyl alcohol, or a combination thereof.

7. The cleansing composition of any of the preceding claims, wherein the fatty acid is neutralized, preferably wherein the fatty acid is neutralized with an oxide, preferably wherein the oxide comprises sodium hydroxide, potassium hydroxide or a combination thereof.

8. The cleansing composition of any of the preceding claims, wherein the organic buffer has a pKa range of 6.0 to 9.0, preferably, 6.5 to 8.5, more preferably, 7.0 to 8.0.

9. The cleansing composition of any of the preceding claims, wherein the organic buffer comprises triethanolamine, diethanolamine, triethanolamine sodium phosphate, calcium carbonate, sodium phosphate, disodium phosphate, trisodium phosphate, or a combination thereof.

10. The cleansing composition of any of the preceding claims, wherein the molar ratio of the organic buffer and surfactant is 1:5 to 3:2, preferably 1:4 to 1:1, more preferably 1:4 to 1:2.

11. The cleansing composition of any of the preceding claims, wherein the molar ratio of the organic buffer to total fatty acid in the cleansing composition is 1.2 to 1.5, preferably, 1.15 to 1.7, more preferably, 1.12 to 1.10.

12. The cleansing composition of any of the preceding claims, wherein the organic buffer is present in an amount of 0.1 to 0.7% by weight.

13. A method of forming a foaming composition, comprising:
forming a composition of any of Claims 1-12; and
agitating the composition for less than or equal to 5 minutes, preferably, less than or equal to 4 minutes, more preferably, less than or equal to 3 minutes to form the foam.

14. The method of Claim 13, wherein the composition forms a measurable amount of lather within 4 seconds constantly.

15. Use of the composition of any of Claims 1-12 in a body cleansing or hair cleansing application.

## Patentansprüche

1. Reinigungsmittelzusammensetzung, umfassend:
weniger als 12 Gewichts-% einer C10-C18-Fettsäure;
0,25 bis 5 Gewichts-% eines Tensids; und
0,1 bis 3 Gewichts-% eines organischen Puffers;
wobei der pH-Wert der Reinigungsmittelzusammensetzung 8,0 bis 10,0, vorzugsweise 8,5 bis 10,0, besonders bevorzugt 9,0 bis 10,0, beträgt.

2. Reinigungsmittelzusammensetzung nach Anspruch 1, wobei die Fettsäure aus Laurinsäure, Myristinsäure, Palmitinsäure, Stearinsäure, Behensäure, Ölsäure, Linolsäure, Lanolinsäure, Isostearinsäure, Arachidonsäure, Hydroxystearinsäure oder einer Kombination davon ausgewählt ist.

3. Reinigungsmittelzusammensetzung nach Anspruch 1 oder Anspruch 2, wobei das Tensid aus einem anionischen Tensid, einem zwitterionischen Tensid, einem amphoteren Tensid oder einer Kombination davon ausgewählt ist.

4. Reinigungsmittelzusammensetzung nach Anspruch 3, wobei das anionische Tensid aus 2-Acrylamido-2-methylpropansulfonsäure, Ammoniumlaurylsulfat, Ammoniumperfluorononanoat, Kaliumlaurylsulfat, Natriumalkylsulfat, Natriumdodecylsulfat, Natriumlaurat, Natriumlaurethsulfat, Natriumlauroylsarcosinat, Natriumstearat, Natriumsulfosuccinatestern oder einer Kombination davon ausgewählt ist.

5. Reinigungsmittelzusammensetzung nach Anspruch 3, wobei das zwitterionische Tensid aus Laurylbetain, Betaincitrat, Natriumhydroxymethylgylcinat, (Carboxymethyl)dimethyl-3-[(1-oxododecyl)amino]propylammoniumhydroxid, Cocalkyldimethylbetainen, (Carboxymethyl)dimethyloleylammoniumhydroxid, Cocamidopropylbetain, (Carboxylatomethyl)dimethyl(octadecyl)ammonium, Cocamidopropyl-hydroxysultain oder einer Kombination davon ausgewählt ist oder wobei das amphotere Tensid aus Natriumlauroamphoacetat, Natriumcocamphoacetat, Natriumlauroamphoacetat, Natriumcocamphoacetat und oder einer Kombination davon ausgewählt ist.

6. Reinigungsmittelzusammensetzung nach Anspruch 3, wobei das Tensid ferner fakultativ ein nichtionisches Tensid umfasst, wobei das nicht-ionische Tensid aus Alkylpolyglycosid, Cetylalkohol, Decylglucosid, Laurylglucosid, Octaethylenglycolmonododecylether, n-Octyl-beta-d-thioglucopyranosid, Octylglucosid, Oleylalkohol, Polysorbat, Sorbitan, Stearylalkohol oder einer Kombination davon ausgewählt ist.

7. Reinigungsmittelzusammensetzung nach einem der vorhergehenden Ansprüche, wobei die Fettsäure neutralisiert ist, wobei die Fettsäure vorzugsweise mit einem Oxid neutralisiert ist, wobei das Oxid vorzugsweise Natriumhydroxid, Kaliumhydroxid oder eine Kombination davon umfasst.

8. Reinigungsmittelzusammensetzung nach einem der vorhergehenden Ansprüche, wobei der organische Puffer einen pKa-Bereich von 6,0 bis 9,0, vorzugsweise von 6,5 bis 8,5, besonders bevorzugt von 7,0 bis 8,0, aufweist.

9. Reinigungsmittelzusammensetzung nach einem der vorhergehenden Ansprüche, wobei der organische Puffer Triethanolamin, Diethanolamin, Triethanolaminnatriumphosphat, Calciumcarbonat, Natriumphosphat, Dinatriumphosphat, Trinatriumphosphat oder eine Kombination davon umfasst.

10. Reinigungsmittelzusammensetzung nach einem der vorhergehenden Ansprüche, wobei das Molverhältnis des organischen Puffers und des Tensids 1:5 bis 3:2, vorzugsweise 1:4 bis 1:1, besonders bevorzugt 1:4 bis 1:2, beträgt.

11. Reinigungsmittelzusammensetzung nach einem der vorhergehenden Ansprüche, wobei das Molverhältnis des organischen Puffers zur gesamten Fettsäure in der Reinigungsmittelzusammensetzung 1,2 bis 1,5, vorzugsweise 1,15 bis 1,7, besonders bevorzugt 1,12 bis 1,10, beträgt.

12. Reinigungsmittelzusammensetzung nach einem der vorhergehenden Ansprüche, wobei der organische Puffer in einer Menge von 0,1 bis 0,7 Gewichts% vorliegt.

13. Verfahren zur Herstellung einer schäumenden Zusammensetzung, umfassend: Herstellen einer Zusammensetzung nach einem der Ansprüche 1-12 und Rühren der Zusammensetzung für weniger als oder gleich 5 Minuten, vorzugsweise weniger als oder gleich 4 Minuten, besonders bevorzugt weniger als oder gleich 3 Minuten, um den Schaum herzustellen.

14. Verfahren nach Anspruch 13, wobei die Zusammensetzung innerhalb von 4 Sekunden konstant eine messbare Schaummenge bildet.

15. Verwendung der Zusammensetzung nach einem der Ansprüche 1-12 in einer Körperreinigungs- oder Haarreinigungsanwendung.

## Revendications

1. Composition de nettoyage, comprenant :
moins de 12 % en masse d'un acide gras en C10-C18 ;
0,25 à 5 % en masse d'un tensioactif ; et
0,1 à 3 % en masse d'un tampon organique ;
dans laquelle un pH de la composition de nettoyage est de 8,0 à 10,0, de préférence de 8,5 à 10,0, encore mieux de 9,0 à 10,0.

2. Composition de nettoyage selon la revendication 1, dans laquelle l'acide gras est choisi parmi l'acide laurique, l'acide myristique, l'acide palmitique, l'acide stéarique, l'acide béhénique, l'acide oléique, l'acide linoléique, l'acide lanolique, l'acide isostéarique, l'acide arachidonique, l'acide hydroxystéarique, ou une combinaison de ceux-ci.

3. Composition de nettoyage selon la revendication 1 ou revendication 2, dans laquelle le tensioactif est choisi parmi un tensioactif anionique, un tensioactif zwitterionique, un tensioactif amphotère, ou une combinaison de ceux-ci.

4. Composition de nettoyage selon la revendication 3, dans laquelle le tensioactif anionique est choisi parmi l'acide 2-acrylamido-2-méthylpropane sulfonique, des esters de lauryl sulfate d'ammonium, perfluorononanoate d'ammonium, lauryl sulfate de potassium, alkyl sulfate de sodium, dodécyl sulfate de sodium, laurate de sodium, laureth sulfate de sodium, lauroyl sarcosinate de sodium, stéarate de sodium, sulfosuccinate de sodium, ou une combinaison de ceux-ci.

5. Composition de nettoyage selon la revendication 3, dans laquelle le tensioactif zwitterionique est choisi parmi la lauryl bétaïne, le citrate de bétaïne, l'hydroxyméthylglycinate de sodium, l'hydroxyde de (carboxyméthyl)diméthyl-3-[(1-oxododécyl)amino]propylammonium, des cocoalkyldiméthyl bétaïnes, l'hydroxyde de (carboxyméthyl)diméthyloléylammonium, la cocoamidopropyl bétaïne, le (carboxylatométhyl)diméthyl(octadécyl)ammonium, la cocamidopropyl hydroxysultaïne, ou une combinaison de ceux-ci ou dans laquelle le tensioactif amphotère est choisi parmi le lauroamphoacétate de sodium, le cocoamphoacétate de sodium, le lauroamphoacétate de sodium, le cocoamphoacétate de sodium ou une combinaison de ceux-ci.

6. Composition de nettoyage selon la revendication 3, dans laquelle le tensioactif comprend de plus éventuellement un tensioactif non-ionique, dans laquelle le tensioactif non-ionique est choisi parmi un alkylpolyglycoside, l'alcool cétylique, le décylglucoside, le laurylglucoside, le monododécyléther d'octaéthylène glycol, le n-octyl bêta-d-thioglucopyranoside, l'octylglucoside, l'alcool oléylique, le polysorbate, le sorbitane, l'alcool stéarylique, ou une combinaison de ceux-ci.

7. Composition de nettoyage selon l'une quelconque des revendications précédentes, dans laquelle l'acide gras est neutralisé, de préférence dans laquelle l'acide gras est neutralisé avec un oxyde, de préférence dans laquelle l'oxyde comprend de l'hydroxyde de sodium, hydroxyde de potassium ou une combinaison de ceux-ci.

8. Composition de nettoyage selon l'une quelconque des revendications précédentes, dans laquelle le tampon organique présente un intervalle de pKa de 6,0 à 9,0, de préférence, 6,5 à 8,5, encore mieux, 7,0 à 8,0.

9. Composition de nettoyage selon l'une quelconque des revendications précédentes, dans laquelle le tampon organique comprend la triéthanolamine, la diéthanolamine, le phosphate de triéthanolamine sodium, le carbonate de calcium, le phosphate de sodium, le phosphate de disodium, le phosphate de trisodium ou une combinaison de ceux-ci.

10. Composition de nettoyage selon l'une quelconque des revendications précédentes, dans laquelle le rapport molaire du tampon organique et tensioactif est de 1:5 à 3:2, de préférence 1:4 à 1:1, encore mieux 1:4 à 1:2.

11. Composition de nettoyage selon l'une quelconque des revendications précédentes, dans laquelle le rapport molaire du tampon organique à acide gras total dans la composition de nettoyage est de 1,2 à 1,5, de préférence, 1,15 à 1,7, encore mieux, 1,12 à 1,10.

12. Composition de nettoyage selon l'une quelconque des revendications précédentes, dans laquelle le tampon organique est présent dans une quantité de 0,1 à 0,7 % en masse.

13. Procédé de formation d'une composition moussante, comprenant :
la formation d'une composition selon l'une quelconque des revendications 1-12 ; et
l'agitation de la composition pendant 5 minutes ou moins, de préférence, 4 minutes ou moins, encore mieux, 3 minutes ou moins pour former la mousse.

14. Procédé selon la revendication 13, dans lequel la composition forme une quantité mesurable de mousse en 4 secondes de manière constante.

15. Utilisation de la composition selon l'une quelconque des revendications 1-12 dans une application de nettoyage du corps ou de nettoyage des cheveux.
